# EUROPEAN PATENT APPLICATION

(11) **EP 4 806 991 A1**
(43) Date of publication of application: **16.09.2026**
(21) Application number: 24904112.0
(22) Date of filing: 29.11.2024
(51) Int. Cl.: C12N 9/10, C12N 15/77, C12P 13/08

(54) **MICROORGANISM HAVING ENHANCED ACTIVITY OF ACETOHYDROXY ACID SYNTHASE LARGE SUBUNIT AND USES THEREOF**

(30) Priority: 14.12.2023 KR 20230181939
(71) Applicant: CJ Cheiljedang Corporation, Seoul 04560 (KR)
(72) Inventor: LEE, Hyein, Seoul 04560 (KR); OH, Haena, Seoul 04560 (KR); YUN, Hyojin, Seoul 04560 (KR); KIM, Seon Hye, Seoul 04560 (KR)
(74) Representative: Meissner Bolte Partnerschaft mbB
(86) International application number: PCT/KR2024/019313
(87) International publication number: WO 2025/127547

(57) **Abstract**

The present invention provides a modified acetohydroxy acid synthase large subunit, and a microorganism into which the modified acetohydroxy acid synthase large subunit is introduced exhibits excellent Valine productivity.

## Description

### [TECHNICAL FIELD]

### Cross-reference to related application(s)

The present disclosure claims the benefit of priority based on Korean Patent Application No. 10-2023-0181939, filed on December 14, 2023, and the entire disclosure thereof is incorporated herein by reference.

The present disclosure relates to a microorganism having enhanced activity of acetohydroxy acid synthase large subunit and a use thereof.

### [BACKGROUND ART]

L-amino acids are basic structural units of proteins and are used as important materials for pharmaceutical raw materials, food additives, animal feed, nutritional supplements, insecticides, fungicides, and the like. In particular, branched-chain amino acids (BCAAs) are a collective term for L-valine, L-leucine, and L-isoleucine, which are essential amino acids, and said branched-chain amino acids are known to have an antioxidant effect and an effect of directly promoting protein synthesis in muscle cells.

On the other hand, the production of branched-chain amino acids using microorganisms is mainly performed using microorganisms of the genus *Corynebacterium*, but the production of branched-chain amino acids using said microorganisms has a problem in that industrial mass production is difficult.

### [Prior Art Documents]

### [Patent Documents]

(Patent Document 1) U.S. Patent No. US 8465962 B2

### [DETAILED DESCRIPTION OF THE INVENTION]

### [TECHNICAL PROBLEM]

An object of the present disclosure is to provide a polypeptide in which an amino acid corresponding to the 150th residue from the N-terminus in the amino acid sequence of SEQ ID NO: 1 is substituted with another amino acid.

Another object of the present disclosure is to provide a polynucleotide encoding the polypeptide.

Another object of the present disclosure is to provide a recombinant vector comprising the polynucleotide.

Another object of the present disclosure is to provide a microorganism comprising at least one selected from the group consisting of the polypeptide, the polynucleotide, and the recombinant vector.

Another object of the present disclosure is to provide a composition for producing valine comprising the microorganism.

Another object of the present disclosure is to provide a method for producing valine, comprising culturing the microorganism in a medium.

Another object of the present disclosure is to provide a method for increasing valine production, comprising culturing the microorganism in a medium.

### [TECHNICAL SOLUTION]

Specifically, this is described as follows. Meanwhile, each description and embodiment disclosed in the present disclosure may also be applied to other descriptions and embodiments. That is, all combinations of various elements disclosed in the present disclosure fall within the scope of the present disclosure. In addition, the scope of the present disclosure should not be construed as being limited by the specific descriptions described below. Furthermore, throughout the present specification, a number of papers and patent documents are referenced and their citations are indicated. The disclosures of the cited papers and patent documents are incorporated herein by reference in their entirety to more clearly explain the level of the technical field to which the present invention belongs and the content of the present invention. In addition, those of ordinary skill in the art will recognize, or be able to ascertain using no more than routine experimentation, many equivalents to the specific aspects of the present disclosure described herein. In addition, such equivalents are intended to be included in the present disclosure.

Hereinafter, the present disclosure will be described in more detail.

### Polypeptide

The present disclosure is intended to provide a variant acetohydroxy acid synthase large subunit and a microorganism comprising the same, and the like, by confirming that a microorganism into which the variant acetohydroxy acid synthase large subunit is introduced has an increased valine production ability compared to the microorganism before modification.

One aspect provides a polypeptide in which an amino acid corresponding to the 150th residue from the N-terminus in the amino acid sequence of SEQ ID NO: 1 is substituted with another amino acid.

The polypeptide may be a polypeptide having the enzymatic activity of acetohydroxy acid synthase large subunit. The acetohydroxy acid synthase large subunit may have an enzymatic activity (e.g., EC:2.2.1.6) of converting pyruvate into (2S)-2-acetolactate. The (2S)-2-acetolactate is a substance corresponding to a precursor of valine in the valine biosynthetic pathway.

The acetohydroxy acid synthase large subunit may be derived from a microorganism of the genus *Corynebacterium*, for example, *Corynebacterium glutamicum*, but is not limited thereto.

In one embodiment, the acetohydroxy acid synthase large subunit may be represented by the amino acid sequence of SEQ ID NO: 1, and the amino acid sequence may be obtained from a known database (e.g., NCBI Reference Sequence: WP_003861427.1).

The polypeptide may be a polypeptide in which an amino acid corresponding to the 150th residue from the N-terminus in the amino acid sequence of SEQ ID NO: 1 is substituted with another amino acid.

The other amino acid may be an amino acid other than histidine, which is the amino acid corresponding to the 150th residue from the N-terminus in the amino acid sequence of SEQ ID NO: 1, that is, arginine, proline, asparagine, lysine, aspartic acid, glutamic acid, serine, threonine, glutamine, cysteine, glycine, alanine, valine, isoleucine, leucine, methionine, phenylalanine, tyrosine, or tryptophan.

In one embodiment, the polypeptide may be a polypeptide in which the amino acid corresponding to the 150th residue from the N-terminus in the amino acid sequence of SEQ ID NO: 1 is substituted with arginine, proline, asparagine, lysine, aspartic acid, glutamic acid, serine, threonine, glutamine, cysteine, glycine, alanine, valine, isoleucine, leucine, methionine, phenylalanine, tyrosine, or tryptophan.

In one embodiment, the polypeptide may be a polypeptide in which the amino acid corresponding to the 150th residue from the N-terminus in the amino acid sequence of SEQ ID NO: 1 is substituted with arginine, proline, or asparagine.

In one embodiment, the polypeptide comprises an amino acid sequence having at least 99.84%, 99.68%, 99.52%, 99.36%, 99.2%, 99.04%, 98.88%, 98.72%, 98.56%, 98.4%, 98.24%, 98.08%, 97.92%, 97.44%, 97.28%, 97.12%, 96.96%, 96.81%, 96.5%, 96%, 95.5%, 95%, 94.5%, 94%, 93.5%, 93%, 92.5%, 92%, 91.5%, 91%, 90.5%, 90%, 89%, 88%, 87%, 86%, 85%, 84%, 83%, 82%, 81%, 80%, 79%, 78%, 77%, 76%, 75%, 74%, 73%, 72%, 71%, or 70% homology or identity with the amino acid sequence of SEQ ID NO: 1, and the polypeptide may be a polypeptide in which the amino acid corresponding to the 150th residue from the N-terminus in the amino acid sequence of SEQ ID NO: 1 is substituted with another amino acid.

In one embodiment, the polypeptide comprises the amino acid sequence of SEQ ID NO: 1, and may be a polypeptide in which the amino acid residue at position 150 from the N-terminus in the amino acid sequence of SEQ ID NO: 1 is substituted with another amino acid.

In one embodiment, the polypeptide comprises the amino acid sequence of SEQ ID NO: 1, and may be a polypeptide in which the amino acid residue at position 150 from the N-terminus in the amino acid sequence of SEQ ID NO: 1 is substituted with an amino acid selected from the group consisting of arginine, proline, asparagine, lysine, aspartic acid, glutamic acid, serine, threonine, glutamine, cysteine, glycine, alanine, valine, isoleucine, leucine, methionine, phenylalanine, tyrosine, and tryptophan.

In one embodiment, the polypeptide comprises the amino acid sequence of SEQ ID NO: 1, and may be a polypeptide in which the amino acid at the 150th residue from the N-terminus in the amino acid sequence of SEQ ID NO: 1 is substituted with an amino acid selected from the group consisting of arginine, proline, and asparagine.

In one specific embodiment, the polypeptide may comprise an amino acid sequence selected from the group consisting of the amino acid sequences of SEQ ID NOs: 3, 13, and 14, or may consist of said amino acid sequence, but is not limited thereto.

In one embodiment, the polypeptide may be a polypeptide comprising an amino acid sequence having a homology or identity of at least 99.84%, 99.68%, 99.52%, 99.36%, 99.2%, 99.04%, 98.88%, 98.72%, 98.56%, 98.4%, 98.24%, 98.08%, 97.92%, 97.44%, 97.28%, 97.12%, 96.96%, 96.81%, 96.5%, 96%, 95.5%, 95%, 94.5%, 94%, 93.5%, 93%, 92.5%, 92%, 91.5%, 91%, 90.5%, 90%, 89%, 88%, 87%, 86%, 85%, 84%, 83%, 82%, 81%, 80%, 79%, 78%, 77%, 76%, 75%, 74%, 73%, 72%, 71%, or 70% with any one amino acid sequence selected from the group consisting of the amino acid sequences of SEQ ID NO: 3, SEQ ID NO: 13, and SEQ ID NO: 14, and in which the amino acid corresponding to the 150th residue from the N-terminus in the amino acid sequence of SEQ ID NO: 1 is substituted with another amino acid.

It is apparent that, if the polypeptide comprises a mutation in which the amino acid corresponding to the 150th residue from the N-terminus in the amino acid sequence of SEQ ID NO: 1 is substituted with another amino acid, even if other amino acid residues except for the amino acid corresponding to the 150th residue are deleted, modified, substituted, or added, the polypeptide may be included in the scope of the present disclosure as long as it exhibits acetohydroxy acid synthase large subunit activity. For example, the polypeptide may have a sequence addition or deletion that does not change the activity of the polypeptide, a naturally occurring mutation, a silent mutation, or a conservative substitution at the N-terminus, C-terminus, and/or internal region of the amino acid sequence of the polypeptide.

The "conservative substitution" means substituting one amino acid with another amino acid having similar structural and/or chemical properties. Such amino acid substitutions may generally occur based on similarity in polarity, charge, solubility, hydrophobicity, hydrophilicity, and/or amphipathic nature of the residues. Typically, a conservative substitution may have little or no effect on the activity of a protein or polypeptide.

The polypeptide may have an activity of increasing the acetohydroxy acid synthase large subunit activity and/or the valine production ability of a microorganism. The acetohydroxy acid synthase large subunit activity may mean an enzymatic activity (e.g., EC:2.2.1.6) of converting pyruvate into (2S)-2-acetolactate as described above.

In the present disclosure, the term "variant polypeptide" refers to a polypeptide in which one or more amino acids are conservatively substituted and/or modified to be different from the amino acid sequence before variation of the variant polypeptide, but whose functions or properties are maintained. Such a variant polypeptide may generally be identified by modifying one or more amino acids in the amino acid sequence of the polypeptide and evaluating the properties of the modified polypeptide. That is, the ability of the variant polypeptide may be increased, unchanged, or decreased compared to the polypeptide before variation. In addition, some variant polypeptides may include variant polypeptides in which one or more parts such as an N-terminal leader sequence or a transmembrane domain are removed. Other variant polypeptides may include variant polypeptides in which a part is removed from the N- and/or C-terminus of a mature protein. The term "variant polypeptide" may be used interchangeably with terms such as variant, modification, variant polypeptide, mutated protein, mutation, and variant (in English expressions, modification, modified polypeptide, modified protein, mutant, mutein, divergent, variant, etc.), and as long as it is a term used with a mutated meaning, it is not limited thereto. In addition, the variant polypeptide may include deletions or additions of amino acids that have minimal impact on the properties and secondary structure of the polypeptide. For example, a signal (or leader) sequence involved in co-translational or post-translational translocation of a protein may be conjugated to the N-terminus of the variant polypeptide. In addition, the variant polypeptide may be conjugated with another sequence or linker so that it may be identified, purified, or synthesized.

For the purpose of the present disclosure, the variant polypeptide may be a polypeptide in which the amino acid corresponding to the 150th residue from the N-terminus in the amino acid sequence of SEQ ID NO: 1 is substituted with another amino acid.

The variant polypeptide may have an increased activity for increasing acetohydroxy acid synthase large subunit activity and/or the valine production ability of a microorganism compared to the polypeptide before mutation (e.g., a polypeptide comprising the amino acid sequence of SEQ ID NO: 1).

### Polynucleotide

Another aspect provides a polynucleotide encoding the polypeptide.

In the present disclosure, the term "polynucleotide" refers to a polymer of nucleotides in which nucleotide monomers are linked in a long chain by covalent bonds, and may refer to a DNA or RNA strand of a certain length or longer.

The polynucleotide may be a polynucleotide encoding a polypeptide in which the amino acid corresponding to the 150th residue from the N-terminus in the amino acid sequence of SEQ ID NO: 1 is substituted with another amino acid. In one embodiment, the polynucleotide may be a polynucleotide in which the codon encoding histidine, which is the amino acid corresponding to the 150th residue from the N-terminus in the amino acid sequence of SEQ ID NO: 1, is substituted with a codon encoding another amino acid selected from the group consisting of arginine, proline, asparagine, lysine, aspartic acid, glutamic acid, serine, threonine, glutamine, cysteine, glycine, alanine, valine, isoleucine, leucine, methionine, phenylalanine, tyrosine, and tryptophan. In one embodiment, the polynucleotide may be a polynucleotide in which the codon encoding the amino acid corresponding to the 150th residue from the N-terminus in the amino acid sequence of SEQ ID NO: 1 is substituted with a codon encoding an amino acid selected from the group consisting of arginine, proline, and asparagine.

It is clearly known in the art which polynucleotide sequence a codon encoding each amino acid includes. The codon may be subjected to various modifications within a range that does not change the amino acid sequence of the polypeptide, in consideration of the codon degeneracy or a codon preferred in an organism in which the polypeptide is to be expressed.

In one embodiment, the polynucleotide may be one encoding a polypeptide in which the amino acid of the 150th residue from the N-terminus in the amino acid sequence of SEQ ID NO: 1 is substituted with another amino acid. The polynucleotide may be a polynucleotide in which a codon corresponding to the 448th to 450th positions from the 5' terminus in the polynucleotide sequence of SEQ ID NO: 2 is substituted with a codon encoding another amino acid.

In one embodiment, the polynucleotide may be a polynucleotide encoding a polypeptide comprising any one amino acid sequence selected from the group consisting of the amino acid sequences of SEQ ID NO: 3, SEQ ID NO: 13, and SEQ ID NO: 14.

In one specific embodiment, the polynucleotide may comprise any one nucleic acid sequence selected from the group consisting of the nucleic acid sequences of SEQ ID NO: 4, SEQ ID NO: 19, and SEQ ID NO: 20, or may consist of the nucleic acid sequence.

The polynucleotide of the present disclosure may include, without limitation, a probe that may be prepared from a known gene sequence, for example, a sequence that may hybridize under stringent conditions with a complementary sequence to the whole or part of the polynucleotide sequence of the present disclosure. The "stringent conditions" refer to conditions that enable specific hybridization between polynucleotides. Such conditions are specifically described in the literature (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, conditions may be enumerated in which polynucleotides having a high homology or identity, such as at least 99.84%, 99.68%, 99.52%, 99.36%, 99.2%, 99.04%, 98.88%, 98.72%, 98.56%, 98.4%, 98.24%, 98.08%, 97.92%, 97.44%, 97.28%, 97.12%, 96.96%, 96.81%, 96.5%, 96%, 95.5%, 95%, 94.5%, 94%, 93.5%, 93%, 92.5%, 92%, 91.5%, 91%, 90.5%, 90%, 89%, 88%, 87%, 86%, 85%, 84%, 83%, 82%, 81%, 80%, 79%, 78%, 77%, 76%, 75%, 74%, 73%, 72%, 71%, or 70% homology or identity, hybridize with each other, while polynucleotides having a lower homology or identity do not hybridize with each other, or washing conditions for a typical Southern hybridization, which are 60°C, 1×SSC, 0.1% SDS, specifically 60°C, 0.1×SSC, 0.1% SDS, more specifically 68°C, 0.1×SSC, 0.1% SDS, at a salt concentration and temperature equivalent thereto, washing once, specifically two to three times.

Hybridization requires that two polynucleotides have complementary sequences, but hybridized polynucleotides may include some mismatches between bases depending on the stringency of hybridization. The term "complementary" is used to describe the relationship between nucleotide bases that may hybridize with each other. For example, with respect to DNA, adenine is complementary to thymine and cytosine is complementary to guanine. Thus, the polynucleotides of the present disclosure may also include isolated nucleic acid fragments complementary to the entire sequence as well as substantially similar nucleic acid sequences.

Specifically, a polynucleotide having homology or identity with the polynucleotide of the present disclosure may be detected using hybridization conditions including a hybridization step at a Tm value of 55°C and using the above-described conditions. In addition, the Tm value may be 60°C, 63°C, or 65°C, but is not limited thereto and may be appropriately adjusted by those skilled in the art according to the purpose.

Appropriate stringency for hybridizing the polynucleotide depends on the length and degree of complementarity of the polynucleotide, and such variables are well known in the art (e.g., J. Sambrook et al., supra).

In the present disclosure, the statement that a polynucleotide (which may be used interchangeably with 'gene') or a polypeptide (which may be used interchangeably with 'protein') 'comprises a specific nucleic acid sequence or amino acid sequence, consists of, or is expressed as a specific nucleic acid sequence or amino acid sequence' may mean that the polynucleotide or polypeptide essentially comprises the specific nucleic acid sequence or amino acid sequence, and may be interpreted as including a 'substantially equivalent sequence' in which insignificant variations (deletion, substitution, modification, and/or addition) are introduced into the specific nucleic acid sequence or amino acid sequence within a range that maintains the original function and/or desired function of the polynucleotide or polypeptide (or as not excluding such insignificant variations).

In the present disclosure, the term "homology" or "identity" refers to the degree of similarity between two given amino acid sequences or nucleotide sequences and may be expressed as a percentage. The terms homology and identity may often be used interchangeably.

The sequence homology or identity of a conserved polynucleotide or polypeptide is determined by a standard alignment algorithm, and a default gap penalty established by the program used may be used in conjunction. Substantially, homologous or identical sequences may generally hybridize with the whole or a part of the sequence under intermediate or high stringency conditions. It is apparent that hybridization also includes hybridization with a polynucleotide containing a general codon or a codon in consideration of codon degeneracy in the polynucleotide.

Whether any two polynucleotide or polypeptide sequences have homology, similarity, or identity may be determined using a known computer algorithm such as the "FASTA" program using default parameters as in, for example, Pearson et al (1988) [Proc. Natl. Acad. Sci. USA 85]: 2444. Alternatively, it may be determined using the Needleman-Wunsch algorithm (Needleman and Wunsch, 1970, J. Mol. Biol. 48: 443-453) as performed in the Needle program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, FASTA (Altschul, [S.] [F.,] [ET AL, J MOLEC BIOL 215]: 403 (1990); Guide to Huge Computers, Martin J. Bishop, [ED.,] Academic Press, San Diego, 1994, and [CARILLO ET AL.] (1988) SIAM J Applied Math 48: 1073)). For example, homology, similarity, or identity may be determined using BLAST of the National Center for Biotechnology Information, or ClustalW.

Homology, similarity, or identity of polynucleotides or polypeptides may be determined by comparing sequence information using a GAP computer program, such as, for example, Needleman et al. (1970), J Mol Biol. 48:443, as known in Smith and Waterman, Adv. Appl. Math (1981) 2:482. In summary, the GAP program may define the homology, similarity, or identity as a value obtained by dividing the number of similarly aligned symbols (i.e., nucleotides or amino acids) by the total number of symbols in the shorter of the two sequences. Default parameters for the GAP program may include (1) a binary comparison matrix (containing a value of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix), as disclosed by Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10, a gap extension penalty of 0.5); and (3) no penalty for end gaps.

In the present disclosure, the term "corresponding to" refers to an amino acid residue at a position listed in a polypeptide, or an amino acid residue similar, identical, or homologous to a residue listed in a polypeptide. Identifying an amino acid at a corresponding position may be determining a specific amino acid of a sequence referring to a specific sequence. As used in the present disclosure, "corresponding region" generally refers to a similar or corresponding position in a related protein or a reference protein.

For example, an arbitrary amino acid sequence may be aligned with SEQ ID NO: 1, and based on this, each amino acid residue of the amino acid sequence may be numbered by referring to the numerical position of the amino acid residue corresponding to the amino acid residue of SEQ ID NO: 1. For example, a sequence alignment algorithm such as those described in the present disclosure may identify a position of an amino acid, or a position where a modification such as substitution, insertion, or deletion occurs, compared to a query sequence (also referred to as a "reference sequence").

For such alignment, for example, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000, Trends Genet. 16: 276-277), and the like may be used, but without being limited thereto, a sequence alignment program known in the art, a pairwise sequence comparison algorithm, and the like may be appropriately used.

Another aspect provides a vector comprising the polynucleotide. The vector may be an insertion vector or an expression vector.

In the present disclosure, the term "vector" means a DNA construct for delivering a target polynucleotide into a suitable host or host cell. In one embodiment, it may include a nucleic acid sequence of a polynucleotide encoding a target polypeptide operably linked to a suitable expression regulatory region (or expression regulatory sequence) so that the target polypeptide may be expressed in a suitable host cell, but is not limited thereto. The regulatory sequence may include a promoter capable of initiating transcription, any operator sequence for regulating transcription, a sequence encoding a suitable mRNA ribosome binding site, and/or a sequence for regulating termination of transcription and/or translation. After being transformed into a suitable host cell, the vector may be maintained independently of the genome of the host cell or may be inserted into the genome of the host cell. For example, the target polynucleotide may be inserted into a chromosome through an insertion vector. The insertion of the polynucleotide into the chromosome may be performed by any method known in the art, for example, homologous recombination, but is not limited thereto.

A vector usable in the present disclosure is not particularly limited as long as it may be replicated in a host cell, and may be selected from all commonly used vectors. Examples of commonly used vectors include plasmids, cosmids, viruses, bacteriophages, and the like, in a natural state or a recombinant state. For example, as the vector, pWE15, M13, MBL3, MBL4, IXII, ASHII, APII, t10, t11, Charon4A, Charon21A, and the like may be used as a phage vector or a cosmid vector, and pBR-based, pUC-based, pBluescriptII-based, pGEM-based, pTZ-based, pCL-based, and pET-based vectors, and the like may be used as a plasmid vector. Specifically, pDC24 (SEQ ID NO: 21), pDCM2, pDZ, pACYC177, pACYC184, pCL, pECCG117, pUC19, pBR322, pMW118, pCC1BAC vectors, and the like may be exemplified, but are not limited thereto.

The vector may further comprise a selection marker for confirming whether the vector is introduced into a transformed cell or whether the vector is inserted into the genome of the transformed cell. The selection marker is for confirming whether a cell is transformed with the vector or whether the polynucleotide is inserted, and may be selected from the group consisting of genes that confer a selectable phenotype such as drug resistance, auxotrophy, resistance to cytotoxic agents, and expression of surface proteins. In an environment treated with a selective agent, only cells expressing the selection marker survive or exhibit other phenotypic traits, so transformed cells may be selected.

Expressing the polypeptide in a microorganism may be performed by introducing a polynucleotide encoding the polypeptide or a vector comprising the polynucleotide into a host cell and culturing a recombinant cell (e.g., a microorganism) comprising the same.

Introduction of the polynucleotide encoding the polypeptide or the vector comprising the polynucleotide into a microorganism may be performed by a person skilled in the art appropriately selecting a known transformation method. In the present disclosure, the term "transformation" means changing the genetic trait of a host cell (microorganism) by introducing a target polynucleotide or a vector comprising the polynucleotide into the host cell (microorganism). The transformed polynucleotide may be located by being inserted into a chromosome of a host cell or may be located outside the chromosome. The polynucleotide may be introduced in an appropriate form according to the purpose of introduction. For example, the polynucleotide may be introduced into a host cell in the form of an expression cassette, which is a gene construct including all elements necessary for self-expression. The expression cassette may typically include expression regulatory elements such as a promoter, a transcription termination signal, a ribosome binding site, and/or a translation termination signal, which are operably linked to the polynucleotide. The expression cassette may be in the form of an expression vector capable of self-replication. In addition, the polynucleotide may be introduced into a host cell in its own form and operably linked to a sequence required for expression in the host cell. In the above, the term "operably linked" may mean that an expression regulatory element (e.g., a promoter) and a polynucleotide are functionally linked to perform transcription regulation (e.g., transcription initiation) of the polynucleotide. Operable linkage may be performed using gene recombination techniques known in the art.

The method of transforming the polynucleotide into a host cell may be performed by any method of introducing a nucleic acid into a cell (microorganism), and a transformation technique known in the art may be appropriately selected and performed according to the host cell. Examples of the known transformation method may include electroporation, calcium phosphate (CaPO₄) precipitation method, calcium chloride (CaCl₂) precipitation method, microinjection, polyethylene glycol (PEG) precipitation method (polyethylene glycol-mediated uptake), DEAE-dextran method, cationic liposome method, lipofection, lithium acetate-DMSO method, and the like, but are not limited thereto.

The polypeptide in which the amino acid corresponding to the 150th residue from the N-terminus in the amino acid sequence of SEQ ID NO: 1 is substituted with another amino acid may be one in which the activity of the acetohydroxy acid synthase large subunit is enhanced.

In the present disclosure, the term "enhancement" of polypeptide activity means that the activity of a polypeptide in a host cell (microorganism) is increased compared to its intrinsic activity. The enhancement may be used interchangeably with terms such as activation, up-regulation, overexpression, and increase. Here, activation, enhancement, up-regulation, overexpression, and increase may all include exhibiting an activity that was not originally possessed, or exhibiting an improved activity compared to the intrinsic activity or the activity before modification. The "intrinsic activity" refers to the activity of a specific polypeptide originally possessed by a parent strain or an unmodified microorganism before phenotypic change in cases where a trait is changed by genetic variation due to natural or artificial factors. This may be used interchangeably with "activity before modification." The expression that the activity of a polypeptide is "enhanced," "up-regulated," "overexpressed," or "increased" compared to the intrinsic activity means that the activity is improved compared to the activity and/or concentration (expression level) of a specific polypeptide originally possessed by a parent strain or an unmodified microorganism before phenotypic change.

The enhancement may be achieved by introducing an exogenous polypeptide or through the enhancement of activity and/or concentration (expression level) of an endogenous polypeptide. Whether the activity of the polypeptide is enhanced may be confirmed from an increase in the degree of activity of the polypeptide, the expression level, or the amount of a product produced from the polypeptide.

Enhancement of the activity of the polypeptide may be performed by applying various methods well known in the art, and the methods are not limited as long as the activity of the target polypeptide may be enhanced compared to that of the microorganism before modification. Specifically, the enhancement may be performed using genetic engineering and/or protein engineering well known to those skilled in the art, which are routine methods in molecular biology, but is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be
1) an increase in the intracellular copy number of a polynucleotide encoding the polypeptide;
2) replacement of an expression regulatory region of a chromosomal gene encoding the polypeptide with a sequence having strong activity;
3) modification of a nucleotide sequence encoding a start codon or a 5'-UTR region of a gene transcript encoding the polypeptide;
4) modification of an amino acid sequence of the polypeptide to enhance the activity of the polypeptide;
5) modification of a polynucleotide sequence encoding the polypeptide to enhance the activity of the polypeptide (for example, modification of a polynucleotide sequence of the polypeptide gene to encode a polypeptide modified to enhance the activity of the polypeptide);
6) introduction of an exogenous polypeptide exhibiting the activity of the polypeptide or an exogenous polynucleotide encoding the same;
7) codon optimization of a polynucleotide encoding the polypeptide;
8) analysis of the tertiary structure of the polypeptide to select an exposed site and modify or chemically modify the same; or
9) a combination of two or more selected from items 1) to 8) above, but is not particularly limited thereto.

More specifically:
The increase in the intracellular copy number of the polynucleotide encoding the polypeptide as described in item 1) may be achieved by introducing into a host cell a vector to which the polynucleotide encoding the corresponding polypeptide is operably linked and which is capable of replicating and functioning independently of the host. Alternatively, it may be achieved by introducing one copy or two or more copies of the polynucleotide encoding the corresponding polypeptide into a chromosome within a host cell. The introduction into the chromosome may be performed by introducing into the host cell a vector capable of inserting the polynucleotide into a chromosome in the host cell, but is not limited thereto. The vector is as described above.

The replacement of a gene expression regulatory region (or an expression regulatory sequence) on a chromosome encoding the polypeptide as described in item 2) with a sequence having strong activity may be, for example, the occurrence of a variation in the sequence by deletion, insertion, non-conservative or conservative substitution, or a combination thereof to further enhance the activity of the expression regulatory region, or replacement with a sequence having stronger activity. The expression regulatory region may include, but is not particularly limited to, a promoter, an operator sequence, a sequence encoding a ribosome binding site, and a sequence regulating the termination of transcription and translation. For example, the replacement may be replacing the original promoter with a strong promoter, but is not limited thereto.

Examples of known strong promoters include, but are not limited to, the CJ1 to CJ7 promoters (U.S. Patent No. 7,662,943 B2), the lac promoter, the trp promoter, the trc promoter, the tac promoter, the lambda phage PR promoter, the PL promoter, the tet promoter, the gapA promoter, the SPL7 promoter, the SPL13(sm3) promoter (U.S. Patent No. 10,584,338 B2), the O2 promoter (U.S. Patent No. 10,273,491 B2), the tkt promoter, and the yccA promoter.

The modification of the nucleotide sequence encoding the start codon or the 5'-UTR region of the gene transcript encoding the polypeptide as described in item 3) above may be, for example, substitution with a nucleotide sequence encoding another start codon having a higher polypeptide expression rate than an endogenous start codon, but is not limited thereto.

The modification of the amino acid sequence or polynucleotide sequence as described in items 4) and 5) above may be the occurrence of a variation in the sequence by deletion, insertion, non-conservative or conservative substitution, or a combination thereof in the amino acid sequence of the polypeptide or the polynucleotide sequence encoding the polypeptide to enhance the activity of the polypeptide, or replacement with an amino acid sequence or polynucleotide sequence improved to have stronger activity or an amino acid sequence or polynucleotide sequence improved to increase activity, but is not limited thereto. Specifically, the replacement may be performed by inserting a polynucleotide into a chromosome by homologous recombination, but is not limited thereto. The vector used in this case may further include a selection marker for confirming whether insertion into the chromosome has occurred. The selection marker is as described above.

The introduction of the exogenous polynucleotide exhibiting the activity of the polypeptide as described in item 6) may be the introduction into a host cell of an exogenous polynucleotide encoding a polypeptide exhibiting activity identical or similar to that of the polypeptide. The exogenous polynucleotide is not limited in its origin or sequence as long as it exhibits activity identical or similar to that of the polypeptide. The method used for the introduction may be performed by a person skilled in the art by appropriately selecting a known transformation method, and a polypeptide may be produced and its activity may be increased by expressing the introduced polynucleotide in the host cell.

The codon optimization of the polynucleotide encoding the polypeptide as described in item 7) above may be a codon optimization of an endogenous polynucleotide to increase transcription or translation in a host cell, or a codon optimization of an exogenous polynucleotide to achieve optimized transcription and translation in a host cell.

The analysis of the tertiary structure of the polypeptide to select an exposed site and modify or chemically modify it as described in 8) may be, for example, determining template protein candidates according to a degree of sequence similarity by comparing sequence information of the polypeptide to be analyzed with a database in which sequence information of known proteins is stored, confirming the structure based on the determined candidates, and then selecting and modifying or chemically modifying an exposed site to be modified or chemically modified.

Such enhancement of polypeptide activity may be one in which the activity or expression level (or concentration) of a corresponding polypeptide is increased based on the activity or concentration of a polypeptide expressed in a wild-type or a microorganism before modification, or an amount of a product produced from the polypeptide is increased, but is not limited thereto.

### Microorganism

Another aspect provides a microorganism comprising at least one selected from the group consisting of the polypeptide, the polynucleotide encoding the polypeptide, and the vector comprising the polynucleotide (e.g., one or more, two or more, or one, two, or three).

In the present disclosure, the term "microorganism (or strain)" may include both wild-type microorganisms and microorganisms in which genetic modification has occurred naturally or artificially. The microorganism is a microorganism in which a specific mechanism is enhanced or weakened due to reasons such as insertion of an external gene or enhancement or weakening of the activity of an endogenous gene, and may be a microorganism including genetic modification for production of a target polypeptide, protein, or product (e.g., valine). In the present disclosure, the terms "microorganism," "strain," "host," and "host cell" may be used interchangeably.

The microorganism (or strain, recombinant cell) of the present disclosure may be a microorganism in which the enzymatic activity of an acetohydroxy acid synthase large subunit is enhanced, a microorganism having valine production ability (or production level), or a microorganism in which valine production ability is enhanced (or increased). The valine may be L-valine.

As an example, the microorganism of the present disclosure may be a microorganism in which valine production ability is conferred or enhanced by introducing at least one selected from the group consisting of the polypeptide, the polynucleotide encoding the polypeptide, and the vector comprising the polynucleotide into a microorganism naturally having no valine production ability or a microorganism having valine production ability, but is not limited thereto.

The phrase that the microorganism has enhanced valine production ability or has valine production ability may mean that the microorganism has an improved valine production ability compared to an unmodified microorganism, a cell before recombination, a parent strain, or a wild-type strain, or that valine production ability is conferred, unlike an unmodified microorganism, a cell before recombination, a parent strain, or a wild-type strain having no valine production ability.

In the present disclosure, "unmodified microorganism" does not exclude a strain including a mutation that may naturally occur in a microorganism, and may mean a wild-type strain or a natural strain itself, or a strain before a trait is changed by genetic variation due to natural or artificial factors. For example, the unmodified microorganism may mean a strain into which the variant polypeptide of the present disclosure or the polynucleotide encoding the variant polypeptide has not been introduced, or a strain prior to the introduction thereof, according to an example. The "unmodified microorganism" may be used interchangeably with "strain before modification," "microorganism before modification," "non-mutant strain," "unmodified strain," "non-mutant microorganism," or "reference microorganism."

The microorganism (or strain, recombinant cell) may additionally comprise a mutation that increases valine production, and the location of the mutation and/or the type of gene and/or protein to be mutated may be included without limitation as long as valine production is increased. The recombinant cell may be used without limitation as long as it is a cell capable of transformation.

In one embodiment, the microorganism of the present disclosure may further include an A42V variant of acetolactate synthase isozyme 1 small subunit (IlvN) protein or a nucleotide encoding the same (refer to Biotechnology and Bioprocess Engineering, June 2014, Volume 19, Issue 3, pp 456-467).

In one embodiment, the target strain for comparing whether valine production ability is increased may be a microorganism belonging to the genus *Corynebacterium*, such as *Corynebacterium glutamicum*, for example, wild-type *Corynebacterium glutamicum* ATCC14067 strain, *Corynebacterium glutamicum* CA08-0072 strain (KCCM11201P, US 8465962 B), a microorganism in which an ilvN A42V mutation is introduced into the *Corynebacterium glutamicum* ATCC14067 strain (ilvN(A42V); Biotechnology and Bioprocess Engineering, June 2014, Volume 19, Issue 3, pp 456-467), and the like, but is not limited thereto.

The microorganism may be a microorganism of the genus *Corynebacterium* (*Corynebacterium* sp.). The microorganism of the genus *Corynebacterium* may be one or more microorganisms selected from the group consisting of *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium pollutisoli, Corynebacterium imitans, Corynebacterium testudinoris*, and *Corynebacterium flavescens*, but is not limited thereto.

For example, the microorganism with enhanced valine production ability may be one in which valine production ability is newly conferred or increased by about 3% or more, about 4% or more, about 5% or more, about 6% or more, about 7% or more, about 8% or more, about 9% or more, about 10% or more, about 11% or more, about 12% or more, about 13% or more, about 14% or more, about 15% or more, about 16% or more, about 17% or more, about 18% or more, about 19% or more, about 20% or more, about 21% or more, about 22% or more, about 23% or more, about 24% or more, about 25% or more, about 30% or more, about 40% or more, about 50% or more, about 60% or more, about 70% or more, about 80% or more, about 90% or more, about 100% or more, about 150% or more, about 200% or more, about 250% or more, about 300% or more, about 400% or more, about 500% or more, about 600% or more, about 700% or more, about 800% or more, about 900% or more, about 1,000% or more, about 1,500% or more, about 2,000% or more, about 2,500% or more, or about 3,000% or more compared to a parent strain before mutation or an unmodified microorganism, but is not limited thereto.

As another example, the microorganism with enhanced valine production ability may have a valine production ability of about 1.03 times or more, about 1.04 times or more, about 1.05 times or more, about 1.06 times or more, about 1.07 times or more, about 1.08 times or more, about 1.9 times or more, about 1.1 times or more, about 1.2 times or more, about 1.3 times or more, about 1.4 times or more, about 1.5 times or more, about 1.6 times or more, about 1.7 times or more, about 1.8 times or more, about 1.9 times or more, about 2 times or more, about 2.5 times or more, about 3 times or more, about 4 times or more, about 5 times or more, about 6 times or more, about 7 times or more, about 8 times or more, about 9 times or more, about 10 times or more, about 15 times or more, about 20 times or more, about 25 times or more, or about 30 times or more (the upper limit is not particularly limited and, for example, may be about 1,000 times or less) compared to a parent strain before mutation or an unmodified microorganism, but is not limited thereto.

As another example, the microorganism with enhanced valine production ability may have a valine production ability increased by about 0.1 g/L or more, about 0.2 g/L or more, about 0.3 g/L or more, about 0.4 g/L or more, about 0.5 g/L or more, about 0.6 g/L or more, about 0.7 g/L or more, about 0.8 g/L or more, about 0.9 g/L or more, about 1 g/L or more, about 1.1 g/L or more, about 1.2 g/L or more, about 1.3 g/L or more, about 1.4 g/L or more, about 1.5 g/L or more, about 1.6 g/L or more, about 1.7 g/L or more, about 1.8 g/L or more, about 1.9 g/L or more, about 2.0 g/L or more, about 2.5 g/L or more, about 3 g/L or more, about 3.5 g/L or more, about 4 g/L or more, about 4.1 g/L or more, about 4.2 g/L or more, about 4.3 g/L or more, about 4.4 g/L or more, about 4.5 g/L or more, about 4.6 g/L or more, about 4.7 g/L or more, about 4.8 g/L or more, about 4.9 g/L or more, about 5 g/L or more, about 5.5 g/L or more, about 6 g/L or more, about 7 g/L or more, about 8 g/L or more, about 9 g/L or more, about 10 g/L or more, about 15 g/L or more, about 20 g/L or more, about 25 g/L or more, or about 30 g/L or more (the upper limit is not particularly limited, and for example, may be about 100 g/L or less) compared to a parent strain before mutation or an unmodified microorganism, but is not limited thereto.

The term "about" refers to a range including all of ±0.5, ±0.4, ±0.3, ±0.2, ±0.1, etc., and includes all numerical values within a range equivalent or similar to the numerical value following the term "about," but is not limited thereto.

### Composition for producing valine and method for producing valine

Another aspect is to provide a composition for producing valine comprising the microorganism, a medium in which the microorganism is cultured, or a combination thereof.

Another aspect provides a use of the microorganism for producing valine.

The composition of the present disclosure may further comprise any suitable excipient commonly used in a composition for producing valine, and such an excipient may be, for example, a preservative, a wetting agent, a dispersing agent, a suspending agent, a buffering agent, a stabilizer, or an isotonic agent, etc., but is not limited thereto.

Another aspect provides a method for producing (or manufacturing) valine, comprising culturing the microorganism in a medium.

Another aspect provides a method for increasing valine production, comprising culturing the microorganism in a medium.

The method for producing valine or the method for increasing valine production of the present disclosure may comprise culturing the microorganism in a medium.

In the present disclosure, "culturing" means growing the microorganism, for example, a *Corynebacterium glutamicum* strain, under appropriately controlled environmental conditions. The culturing process may be performed according to suitable media and culture conditions known in the art. Such a culturing process may be easily adjusted and used by those skilled in the art according to the selected strain. Specifically, the culturing may be batch, continuous, and/or fed-batch, but is not limited thereto.

In the present disclosure, "medium" refers to a substance in which nutrients required for culturing the microorganism, for example, a *Corynebacterium glutamicum* strain, are mixed as main components, and the medium supplies nutrients and growth factors, including water essential for survival and growth. Specifically, any medium and other culture conditions used for culturing the microorganism of the present disclosure may be used without particular limitation as long as the medium is one used for culturing a typical microorganism, but the microorganism of the present disclosure may be cultured while adjusting temperature, pH, etc. under aerobic conditions in a typical medium containing an appropriate carbon source, nitrogen source, phosphorus source, inorganic compound, amino acid and/or vitamin, etc.

Specifically, culture media for the microorganism, for example, a strain of the genus *Corynebacterium*, may be found in ["Manual of Methods for General Bacteriology" by the American Society for Bacteriology (Washington D.Corynebacterium, USA, 1981)].

In the present disclosure, the carbon source may include carbohydrates such as glucose, saccharose, lactose, fructose, sucrose, and maltose; sugar alcohols such as mannitol and sorbitol; organic acids such as pyruvate, lactic acid, and citric acid; and amino acids such as glutamic acid, methionine, and lysine. In addition, natural organic nutrients such as starch hydrolysate, molasses (e.g., blackstrap molasses), rice bran, cassava, sugarcane bagasse, and corn steep liquor may be used, and specifically, carbohydrates such as glucose and sterilized pretreated molasses (i.e., molasses converted to reducing sugars) may be used, and various other appropriate amounts of carbon sources may be used without limitation. These carbon sources may be used alone or in combination of two or more, but are not limited thereto.

As the nitrogen source, inorganic nitrogen sources such as ammonia, ammonium sulfate, ammonium chloride, ammonium acetate, ammonium phosphate, ammonium carbonate, ammonium nitrate, and the like, amino acids such as glutamic acid, methionine, glutamine, and the like, and organic nitrogen sources such as peptone, NZ-amine, meat extract, yeast extract, malt extract, corn steep liquor, casein hydrolysate, fish or a decomposition product thereof, defatted soybean cake or a decomposition product thereof, and the like may be used. These nitrogen sources may be used alone or in combination of two or more, but are not limited thereto.

As the phosphorus source, monopotassium phosphate, dipotassium phosphate, or a sodium-containing salt corresponding thereto, and the like may be included. As the inorganic compounds, sodium chloride, calcium chloride, iron chloride, magnesium sulfate, iron sulfate, manganese sulfate, calcium carbonate, and the like may be used, and in addition, amino acids, vitamins and/or appropriate precursors, and the like may be included. These components or precursors may be added to the medium in a batch or continuous manner, but are not limited thereto.

Further, during the culture of the microorganism, compounds such as ammonium hydroxide, potassium hydroxide, ammonia, phosphoric acid, sulfuric acid, and the like may be added to the medium in an appropriate manner to adjust the pH of the medium. In addition, an antifoaming agent such as fatty acid polyglycol ester may be used during the culture to suppress foam formation. Further, in order to maintain the aerobic condition of the medium, oxygen or an oxygen-containing gas may be injected into the medium, or in order to maintain anaerobic and microaerobic conditions, no gas may be injected or nitrogen, hydrogen, or carbon dioxide gas may be injected, but it is not limited thereto.

In the culturing according to the present disclosure, the culture temperature may be maintained at 20 to 45°C, specifically 25 to 40°C, and the culturing may be performed for about 10 to 160 hours, but is not limited thereto.

Valine produced by the culturing of the present disclosure may be secreted into the medium or remain within the cells.

The method for producing valine or the method for increasing valine production of the present disclosure may further comprise, for example, prior to the culturing step, a step of preparing the microorganism, a step of preparing a medium for culturing the microorganism, or a combination thereof (in any order).

The method for producing valine or the method for increasing valine production of the present disclosure may further comprise a step of recovering valine from the medium (the medium in which the culturing was performed) or the microorganism (for example, a strain of the genus *Corynebacterium*) according to the culturing. The step of recovering may be further included after the step of culturing.

The recovery may be collecting the desired valine using a suitable method known in the art according to the culture method of the microorganism of the present disclosure, for example, a batch, continuous or fed-batch culture method, and the like. For example, centrifugation, filtration, crystallization, treatment with a protein precipitant (salting-out), extraction, ultrasonic disruption, ultrafiltration, dialysis, various types of chromatography such as molecular sieve chromatography (gel filtration), adsorption chromatography, ion-exchange chromatography, and affinity chromatography, HPLC, or a combination of these methods may be used, and the desired valine may be recovered from the medium or the microorganism using a suitable method known in the art.

In addition, the method for producing valine or the method for increasing valine production of the present disclosure may additionally comprise a purification step. The purification may be performed using a suitable method known in the art. In one embodiment, when the method for producing valine or the method for increasing valine production of the present disclosure comprises both a recovery step and a purification step, the recovery step and the purification step may be performed continuously or discontinuously in any order, or may be performed simultaneously or integrated into one step, but are not limited thereto.

### [ADVANTAGEOUS EFFECTS]

The present disclosure provides a variant acetohydroxy acid synthase large subunit, and a microorganism into which the variant acetohydroxy acid synthase large subunit is introduced has excellent valine production ability.

### [MODE FOR INVENTION]

Hereinafter, the present invention will be described in more detail with reference to the following examples. However, these examples are intended to illustrate the present invention only, and the scope of the present invention is not limited by these examples.

### Example 1. Screening of mutant strains with increased valine production ability through an artificial mutagenesis method

### Example 1-1. Induction of artificial mutations using a gamma irradiation method

In order to select mutant strains with increased valine production ability, mutations were induced in microorganisms using the following method. Gamma irradiation, which is a physical method, was applied for mutagenesis.

In order to obtain a microorganism with high L-valine-production ability, a gamma irradiation-based mutant library was constructed for strain KCCM11201P (US 8,465,962 B), and high L-valine-producing mutants were screened. To perform gamma irradiation on the KCCM11201P strain, a 500 mL flask containing 50 mL of seed medium was cultured in a shaking incubator at 30°C for 24 hours to obtain a culture solution having an absorbance value of 7.84 at 562 nm, and the culture solution was then diluted with the seed medium to an absorbance value of 6.10 at 562 nm. After irradiating 30 mL of the diluted solution with gamma rays for 1 hour using a high-intensity gamma irradiation device located at the Advanced Radiation Research Institute of the Korea Atomic Energy Research Institute, 100 µl of the undiluted irradiated solution was spread on activation medium in each of 100 90 x 15 mm Petri dishes. The Petri dishes were cultured in a stationary incubator at 30°C for 48 hours to obtain a total of approximately 40,000 individual colonies. To ensure the stability of the mutants, all individual colonies generated in all Petri dishes were recovered and suspended in a 20% glycerol solution, then divided into 1 mL aliquots in 1.5 mL microtubes and stored in an ultra-low temperature freezer at -80°C.

### Example 1-2. Fermentation titer evaluation of mutant strains and strain screening

When screening for mutants with high L-valine-production ability, a 20% glycerol suspension, which was removed from an ultra-low temperature freezer and thawed at room temperature, was serially diluted to 10⁻¹, 10⁻², 10⁻³, 10⁻⁴, and 10⁻⁵ using saline, and 100 µl of the diluted solution corresponding to each dilution factor was spread on an activation medium and reactivated in the form of individual colonies through culturing in a stationary incubator at 30°C for 24 hours. The reactivated individual colonies were inoculated into a 96-deep well plate, in which 350 µl of production medium corresponding to a filling rate of 17% per well was dispensed, using a colony picker (Molecular Devices, Qpix420) facility, and the KCCM11201P strain not irradiated with gamma rays was inoculated into four wells per plate on the same plate and used as a control for screening mutants with high L-valine production ability. The 96-deep well plates inoculated with the control strain and the mutants, respectively, were sealed using a gas-permeable seal mark2 (Azenta) and cultured at 30°C and 1,000 rpm in a shaking incubator (Infors-HT, Multitron) for 48 hours. The 96-deep well plates cultured for 48 hours were subjected to centrifugation at 15°C and 4,000 rpm for 20 minutes in a centrifuge (Eppendorf, Centrifuge 5810R), and then the culture supernatant from which the cells were separated was transferred to a 96-well black polystyrene microplate (Corning) using a liquid handler (Beckman Coulter, Biomek i5) for NIR spectrometry analysis. Thereafter, individual analysis spectra of each well were obtained by applying the samples to a custom-built NIR spectrometer, and 21 strains with excellent L-valine concentration were primarily selected from among 10,304 mutant strains. Thereafter, culturing was performed in the same manner as above for the selected 21 strains (C1 to C21), and then the concentration of L-valine was analyzed; the analyzed L-valine concentrations are shown in Table 1 below.

### [Nutrient medium (pH 7.2)]

10 g of glucose, 5 g of meat extract, 10 g of polypeptone, 2.5 g of sodium chloride, 5 g of yeast extract, 20 g of agar, and 2 g of urea (based on 1 liter of distilled water)

### [Production medium (pH 7.0)]

100 g of glucose, 40 g of ammonium sulfate, 2.5 g of soy protein, 5 g of corn steep solids, 3 g of urea, 1 g of potassium phosphate dibasic, 0.5 g of magnesium sulfate heptahydrate, 100 µg of biotin, 1 mg of thiamine-HCl, 2 mg of calcium pantothenate, 3 mg of nicotinamide, and 30 g of calcium carbonate (based on 1 liter of distilled water)

**[Table 1]**

| | Strain Name | L-valine (g/L) |
|---|---|---|
| Control | KCCM11201P | 2.5 |
| Experimental group | C1 | 2.5 |
| | C2 | 2.9 |
| | C3 | 2.3 |
| | C4 | 2.6 |
| | C5 | 2.3 |
| | C6 | 1.9 |
| | C7 | 2.2 |
| | C8 | 3.0 |
| | **C9** | **4.2** |
| | C10 | 3.1 |
| | C11 | 3.6 |
| | C12 | 3.2 |
| | C13 | 3.5 |
| | C14 | 2.8 |
| | C15 | 2.2 |
| | C16 | 3.3 |
| | C17 | 2.2 |
| | C18 | 2.5 |
| | C19 | 2.7 |
| | C20 | 2.4 |
| | C21 | 3.0 |

Referring to Table 1 above, the C9 strain, which exhibited the greatest increase in valine production compared to the control strain KCCM11201P, was selected.

### Example 2. Confirmation of mutation through gene sequencing

The major genes of the strain were sequenced and compared with the KCCM11201P strain and the *Corynebacterium glutamicum* ATCC14067 wild-type strain. As a result, it was confirmed that the strain with increased valine production ability contains a nucleotide sequence mutation at a specific position in the ORF (open reading frame) region of the ilvB gene. Specifically, it was confirmed that in the C9 strain, which exhibited the greatest increase in valine production, one mutation was introduced into the nucleotide sequence located 449 bp upstream from the start codon of the ilvB gene, resulting in a change from the existing CAC (SEQ ID NO: 2) to CGC (SEQ ID NO: 4), whereby the 150th amino acid histidine (SEQ ID NO: 1) was substituted with arginine (SEQ ID NO: 3).

As a result of analyzing the mutation region of SEQ ID NO: 3, the region was confirmed to affect the effector binding domain of the valine biosynthetic enzyme, and thus the activity of the protein was expected to be enhanced. In the following examples, it was intended to confirm whether the application of the H150R mutation introduced at a specific position of the ORF in the ilvB gene affects the valine production ability of a microorganism of the genus *Corynebacterium*. Further, it was intended to confirm whether substitution with an amino acid other than arginine at the 150th amino acid histidine position also affects the valine production ability of a microorganism of the genus *Corynebacterium*.

### Example 3. Construction of KCCM11201P strain into which an ilvB mutation was introduced and confirmation of valine production ability

### Example 3-1. Preparation of a strain into which an ilvB mutation was introduced in Corynebacterium glutamicum KCCM11201P and evaluation of L-valine production ability

In order to insert the ilvB(H150R) variant represented by SEQ ID NO: 3 into *Corynebacterium glutamicum* KCCM11201P, a vector containing the target mutation was prepared. Specifically, genomic DNA of the C9 strain was extracted using a G-spin Total DNA extraction mini kit (Intron, Cat. No. 17045) according to the protocol provided in the kit, and PCR was performed using the genomic DNA as a template. The PCR was performed under conditions of denaturation at 94°C for 5 minutes; 25 cycles of denaturation at 94°C for 30 seconds, annealing at 55°C for 30 seconds, and extension at 72°C for 150 seconds; and a final extension at 72°C for 7 minutes. An 808 bp PCR product (hereinafter, referred to as "mutation-introduced fragment 1") was obtained using a primer pair of SEQ ID NO: 5 and SEQ ID NO: 6.

The obtained mutation-introduced fragment 1 was linked to a pDC24 vector (SEQ ID NO: 21, Table 2) treated with restriction enzyme SmaI (New England Biolabs, Beverly, MA) using an In-Fusion Cloning Kit (Takara Bio Inc., Otsu, Japan), and then transformed into E. coli DH5α (Invitrogen). After the prepared construct was transformed into E. coli DH5α, the transformants were selected on kanamycin-containing LB medium, and DNA was obtained using a DNA-spin plasmid DNA purification kit (iNtRON) to prepare the vector pDC24-ilvB(H150R) containing the mutation-introduced fragment 1.

**[Table 2]**

| |
|---|
| GTTCGCTTGCTGTCCATAAAACCGCCCAGTCTAGCTATCGCCATGTAAGCCCACTGC AAGCTACCTGCTTTCTCTTTGCGCTTGCGTTTTCCCTTGTCCAGATAGCCCAGTAGCT GACATTCATCCGGGGTCAGCACCGTTTCTGCGGACTGGCTTTCTACGTGTTCCGCTT CCTTTAGCAGCCCTTGCGCCCTGAGTGCTTGCGGCAGCGTGAAGCTAGCTTTTATC GCCATTCGCCATTCAGGCTGCGCAACTGTTGGGAAGGGCGATCGGTGCGGGCCTC TTCGCTATTACGCCAGCTGGCGAAAGGGGGATGTGCTGCAAGGCGATTAAGTTGGG TAACGCCAGGGTTTTCCCAGTCACGACGTTGTAAAACGACGGCCAGTGAATTCGAG CTCGGTACCCGGGGATCCTCTAGAGTCGACCTGCAGGCATGCAAGCTTGGCGTAAT CATGGTCATAGCTGTTTCCTGTGTGAAATTGTTATCCGCTCACAATTCCACACAACATA CGAGCCGGAAGCATAAAGTGTAAAGCCTGGGGTGCCTAATGAGTGAGCTAACTCAC ATTAATTGCGTTGCGCTCACTGCCCGCTTTCCAGTCGGGAAACCTGTCGTGCCAGCT GCATTAATGAATCGGCCAACGCGCGGGGAGAGGCGGTTTGCGTATTGGGCGCTCTT CCGCTTCCTCGCTCACTGACTCGCTGCGCTCGGTCGTTCGGCTGCGGCGAGCGGT ATCAGCTCACTCAAAGGCGGTAATACGGTTATCCACAGAATCAGGGGATAACGCAGG AAAGAACATGTGAGCAAAAGGCCAGCAAAAGGCCAGGAACCGTAAAAAGGCCGCGT TGCTGGCGTTTTTCCATAGGCTCCGCCCCCCTGACGAGCATCACAAAAATCGACGCT CAAGTCAGAGGTGGCGAAACCCGACAGGACTATAAAGATACCAGGCGTTTCCCCCT GGAAGCTCCCTCGTGCGCTCTCCTGTTCCGACCCTGCCGCTTACCGGATACCTGTC CGCCTTTCTCCCTTCGGGAAGCGTGGCGCTTTCTCAATGCTCACGCTGTAGGTATCT CAGTTCGGTGTAGGTCGTTCGCTCCAAGCTGGGCTGTGTGCACGAACCCCCCGTTC AGCCCGACCGCTGCGCCTTATCCGGTAACTATCGTCTTGAGTCCAACCCGGTAAGAC ACGACTTATCGCCACTGGCAGCAGCCACTGGTAACAGGATTAGCAGAGCGAGGTAT GTAGGCGGTGCTACAGAGTTCTTGAAGTGGTGGCCTAACTACGGCTACACTAGAAG GACAGTATTTGGTATCTGCGCTCTGCTGAAGCCAGTTACCTTCGGAAAAAGAGTTGG TAGCTCTTGATCCGGCAAACAAACCACCGCTGGTAGCGGTGGTTTTTTTGTTTGCAA GCAGCAGATTACGCGCAGAAAAAAAGGATCTCAAGAAGATCCTTTGATCTTTTCTACG GGGTCTGACGCTCAGTGGAACGAAAACTCACGTTAAGGGATTTTGGTCATGAGATTA TCAAAAAGGATCTTCACCTAGATCCTTTTGGGGTGGGCGAAGAACTCCAGCATGAGA TCCCCGCGCTGGAGGATCATCCAGCCCTGATAGAAACAGAAGCCACTGGAGCACCT CAAAAACACCATCATACACTAAATCAGTAAGTTGGCAGCATCACCCGACGCACTTTGC GCCGAATAAATACCTGTGACGGAAGATCACTTCGCAGAATAAATAAATCCTGGTGTCC CTGTTGATACCGGGAAGCCCTGGGCCAACTTTTGGCGAAAATGAGACGTTGATCGG CACGTAAGAGGTTCCAACTTTCACCATAATGAAATAAGATCACTACCGGGCGTATTTT TTGAGTTATCGAGATTTTCAGGAGCTGATAGAAACAGAAGCCACTGGAGCACCTCAA AAACACCATCATACACTAAATCAGTAAGTTGGCAGCATCACCCGACGCACTTTGCGC |
| CGAATAAATACCTGTGACGGAAGATCACTTCGCAGAATAAATAAATCCTGGTGTCCCT GTTGATACCGGGAAGCCCTGGGCCAACTTTTGGCGAAAATGAGACGTTGATCGGCA CGTAAGAGGTTCCAACTTTCACCATAATGAAATAAGATCACTACCGGGCGTATTTTTT GAGTTATCGAGATTTTCAGGAGCTCTTTGGCATCGTCTCTCGCCTGTCCCCTCAGTT CAGTAATTTCCTGCATTTGCCTGTTTCCAGTCGGTAGATATTCCACAAAACAGCAGGG AAGCAGCGCTTTTCCGCTGCATAACCCTGCTTCGGGGTCATTATAGCGATTTTTTCG GTATATCCATCCTTTTTCGCACGATATACAGGATTTTGCCAAAGGGTTCGTGTAGACTT TCCTTGGTGTATCCAACGGCGTCAGCCGGGCAGGATAGGTGAAGTAGGCCCACCCG CGAGCGGGTGTTCCTTCTTCACTGTCCCTTATTCGCACCTGGCGGTGCTCAACGGG AATCCTGCTCTGCGAGGCTGGCCGGCTACCGCCGGCGTAACAGATGAGGGCAAGC GGATGGCTGATGAAACCAAGCCAACCAGGAAGGGCAGCCCACCTATCAAGGTGTAC TGCCTTCCAGACGAACGAAGAGCGATTGAGGAAAAGGCGGCGGCGGCCGGCATGA GCCTGTCGGCCTACCTGCTGGCCGTCGGCCAGGGCTACAAAATCACGGGCGTCGT GGACTATGAGCACGTCCGCGAGGGCGTCCCGGAAAACGATTCCGAAGCCCAACCTT TCATAGAAGGCGGCGGTGGAATCGAAATCTCGTGATGGCAGGTTGGGCGTCGCTTG GTCGGTCATTTCGAAAAAGGTTAGGAATACGGTTAGCCATTTGCCTGCTTTTATATAGT TCATATGGGATTCACCTTTATGTTGATAAGAAATAAAAGAAAATGCCAATAGGATATCG GCATTTTCTTTTGCGTTTTTATTTGTTAACTGTTAATTGTCCTTGTTCAAGGATGCTGT CTTTGACAACAGATGTTTTCTTGCCTTTGATGTTCAGCAGGAAGCTCGGCGCAAACG TTGATTGTTTGTCTGCGTAGAATCCTCTGTTTGTCATATAGCTTGTAATCACGACATTG TTTCCTTTCGCTTGAGGTACAGCGAAGTGTGAGTAAGTAAAGGTTACATCGTTAGGC GGATCAAGATCCATTTTTAACACAAGGCCAGTTTTGTTCAGCGGCTTGTATGGGCCA GTTAAAGAATTAGAAACATAACCAAGCATGTAAATATCGTTAGACGTAATGCCGTCAAT CGTCATTTTTGATCCGCGGGAGTCAGTGAACAGGTACCATTTGCCGTTCATTTTAAAG ACGTTCGCGCGTTCAATTTCATCTGTTACTGTGTTAGATGCAATCAGCGGTTTCATCA CTTTTTTCAGTGTGTAATCATCGTTTAGCTCAATCATACCGAGAGCGCCGTTTGCTAA CTCAGCCGTGCGTTTTTTATCGCTTTGCAGAAGTTTTTGACTTTCTTGACGGAAGAAT GATGTGCTTTTGCCATAGTATGCTTTGTTAAATAAAGATTCTTCGCCTTGGTAGCCATC TTCAGTTCCAGTGTTTGCTTCAAATACTAAGTATTTGTGGCCTTTATCTTCTACGTAGT GAGGATCTCTCAGCGTATGGTTGTCGCCTGAGCTGTAGTTGCCTTCATCGATGAACT GCTGTACATTTTGATACGTTTTTCCGTCACCGTCAAAGATTGATTTATAATCCTCTACA CCGTTGATGTTCAAAGAGCTGTCTGATGCTGATACGTTAACTTGTGCAGTTGTCAGT GTTTGTTTGCCGTAATGTTTACCGGAGAAATCAGTGTAGAATAAACGGATTTTTCCGT CAGATGTAAATGTGGCTGAACCTGACCATTCTTGTGTTTGGTCTTTTAGGATAGAATC ATTTGCATCGAATTTGTCGCTGTCTTTAAAGACGCGGCCAGCGTTTTTCCAGCTGTC |
| AATAGAAGTTTCGCCGACTTTTTGATAGAACATGTAAATCGATGTGTCATCCGCATTTT TAGGATCTCCGGCTAATGCAAAGACGATGTGGTAGCCGTGATAGTTTGCGACAGTGC CGTCAGCGTTTTGTAATGGCCAGCTGTCCCAAACGTCCAGGCCTTTTGCAGAAGAG ATATTTTTAATTGTGGACGAATCAAATTCAGAAACTTGATATTTTTCATTTTTTTGCTGTT CAGGGATTTGCAGCATATCATGGCGTGTAATATGGGAAATGCCGTATGTTTCCTTATAT GGCTTTTGGTTCGTTTCTTTCGCAAACGCTTGAGTTGCGCCTCCTGCCAGCAGTGC GGTAGTAAAGGTTAATACTGTTGCTTGTTTTGCAAACTTTTTGATGTTCATCGTTCATG TCTCCTTTTTTATGTACTGTGTTAGCGGTCTGCTTCTTCCAGCCCTCCTGTTTGAAGA TGGCAAGTTAGTTACGCACAATAAAAAAAGACCTAAAATATGTAAGGGGTGACGCCAA AGTATACACTTTGCCCTTTACACATTTTAGGTCTTGCCTGCTTTATCAGTAACAAACCC GCGCGATTTACTTTTCGACCTCATTCTATTAGACTCTCGTTTGGATTGCAACTGGTCT ATTTTCCTCTTTTGTTTGATAGAAAATCATAAAAGGATTTGCAGACTACGGGCCTAAAG AACTAAAAAATCTATCTGTTTCTTTTCATTCTCTGTATTTTTTATAGTTTCTGTTGCATGG GCATAAAGTTGCCTTTTTAATCACAATTCAGAAAATATCATAATATCTCATTTCACTAAAT AATAGTGAACGGCAGGTATATGTGATGGGTTAAAAAGGATCACCCCAGAGTCCCGCT CAGAAGAACTCGTCAAGAAGGCGATAGAAGGCGATGCGCTGCGAATCGGGAGCGG CGATACCGTAAAGCACGAGGAAGCGGTCAGCCCATTCGCCGCCAAGCTCTTCAGCA ATATCACGGGTAGCCAACGCTATGTCCTGATAGCGGTCCGCCACACCCAGCCGGCC ACAGTCGATGAATCCAGAAAAGCGGCCATTTTCCACCATGATATTCGGCAAGCAGGC ATCGCCATGGGTCACGACGAGATCCTCGCCGTCGGGCATCCGCGCCTTGAGCCTG GCGAACAGTTCGGCTGGCGCGAGCCCCTGATGCTCTTCGTCCAGATCATCCTGATC GACAAGACCGGCTTCCATCCGAGTACGTGCTCGCTCGATGCGATGTTTCGCTTGGT GGTCGAATGGGCAGGTAGCCGGATCAAGCGTATGCAGCCGCCGCATTGCATCAGCC ATGATGGATACTTTCTCGGCAGGAGCAAGGTGAGATGACAGGAGATCCTGCCCCGG CACTTCGCCCAATAGCAGCCAGTCCCTTCCCGCTTCAGTGACAACGTCGAGACAGC TGCGCAAGGAACGCCCGTCGTGGCCAGCCACGATAGCCGCGCTGCCTCGTCTTGG AGTTCATTCAGGGCACCGGACAGGTCGGTCTTGACAAAAAGAACCGGGCGCCCCT GCGCTGACAGCCGGAACACGGCGGCATCAGAGCAGCCGATTGTCTGTTGTGCCCA GTCATAGCCGAATAGCCTCTCCACCCAAGCGGCCGGAGAACCTGCGTGCAATCCAT CTTGTTCAATCATGCGAAACGATCCTCATCCTGTCTCTTGATCAGATCTTGATCCCCT GCGCCATCAGATCCTTGGCGGCAAGAAAGCCATCCAGTTTACTTTGCAGGGCTTCC CAACCTTACCAGAGGGCGCCCCAGCTGGCAATTCCG (SEQ ID NO: 21) |

**[Table 3]**

| Primer | Nucleotide sequence |
|---|---|
| SEQ ID NO: 5 | AGCAACCGGCTACGCGCAGGTTACT |
| SEQ ID NO: 6 | AACCTCAACCTGCTTGATCTTGCC |

The pDC24-ilvB(H150R) was transformed into *Corynebacterium glutamicum* KCCM11201P by homologous recombination on the chromosome (van der Rest et al., Appl Microbiol Biotechnol 52:541-545, 1999). A strain in which the vector was inserted into the chromosome by recombination of homologous sequences was selected on a medium containing 25 mg/I of kanamycin. Thereafter, for the transformed *Corynebacterium glutamicum* strain in which the secondary recombination was completed, a strain in which histidine was substituted with arginine at amino acid position 150 of SEQ ID NO: 1 within the ORF of the ilvB gene on the chromosome was confirmed through PCR using a primer pair of SEQ ID NO: 5 and SEQ ID NO: 6. The recombinant strain was named *Corynebacterium glutamicum* KCCM11201P::ilvB(H150R).

In order to compare the valine production ability of the valine-producing strains *Corynebacterium glutamicum* KCCM11201P and KCCM11201P::ilvB(H150R), a flask evaluation was performed. After each strain was subcultured in a nutrient medium, each strain was inoculated into a 250 ml corner-baffled flask containing 25 ml of a production medium and shake-cultured at 30°C and 200 rpm for 72 hours. Thereafter, the concentration of L-valine was analyzed using HPLC, and the analyzed L-valine concentrations are shown in Table 4 below.

### [Nutrient medium (pH 7.2)]

10 g of glucose, 5 g of meat extract, 10 g of polypeptone, 2.5 g of sodium chloride, 5 g of yeast extract, 20 g of agar, and 2 g of urea (based on 1 liter of distilled water)

### [Production medium (pH 7.0)]

100 g of glucose, 40 g of ammonium sulfate, 2.5 g of soy protein, 5 g of corn steep solids, 3 g of urea, 1 g of potassium phosphate dibasic, 0.5 g of magnesium sulfate heptahydrate, 100 µg of biotin, 1 mg of thiamine-HCl, 2 mg of calcium pantothenate, 3 mg of nicotinamide, and 30 g of calcium carbonate (based on 1 liter of distilled water)

**[Table 4]**

| L-valine production ability of KCCM11201P and KCCM11201P::ilvB(H150R) | | | | |
|---|---|---|---|---|
| Strain | L-valine (g/L) | | | |
| | Batch 1 | Batch 2 | Batch 3 | Average |
| KCCM11201P | 2.8 | 2.6 | 2.7 | 2.7 |
| KCCM11201P::*ilvB*(H150R) | 3.3 | 3.1 | 3.3 | 3.2 |

As a result, it was confirmed that the L-valine production ability of the KCCM11201P::ilvB(H150R) strain increased by 19% compared to the control (KCCM11201P).

### Example 3-2: Preparation of a strain into which an ilvN mutation is introduced in the Corynebacterium glutamicum CJ7V strain and evaluation of L-valine production ability

In order to confirm whether an effect of increasing L-valine production ability is also exhibited in other strains belonging to *Corynebacterium glutamicum* that produce L-valine, a strain with improved L-valine production ability was constructed by introducing one mutation [ilvN(A42V); Biotechnology and Bioprocess Engineering, June 2014, Volume 19, Issue 3, pp 456-467] into the wild-type *Corynebacterium glutamicum* ATCC14067.

Specifically, genomic DNA was extracted from the wild-type *Corynebacterium glutamicum* strain ATCC14067 using a G-spin Total DNA extraction mini kit (Intron, Cat. No. 17045) according to the protocol provided in the kit. PCR was performed using the genomic DNA as a template. In order to prepare a vector for introducing an A42V mutation into the ilvN gene, gene fragments (A, B) were obtained using a primer pair of SEQ ID NOs: 7 and 8 and a primer pair of SEQ ID NOs: 9 and 10, respectively. The PCR conditions consisted of denaturation at 94°C for 5 minutes; 25 cycles of denaturation at 94°C for 30 seconds, annealing at 55°C for 30 seconds, and polymerization at 72°C for 60 seconds; and a final polymerization at 72°C for 7 minutes.

As a result, polynucleotides of 528 bp and 509 bp for fragments A and B, respectively, were obtained. Using the two fragments as templates, overlapping PCR was performed using a primer pair of SEQ ID NO: 6 and SEQ ID NO: 9 to obtain a 1010 bp PCR product (hereinafter referred to as "mutation-introduced fragment 2").

The obtained mutation-introduced fragment 2 was treated with restriction enzyme SmaI (New England Biolabs, Beverly, MA), and then ligated with a pDC24 vector treated with the same restriction enzyme by using T4 ligase (New England Biolabs, Beverly, MA). After the prepared construct was transformed into E. coli DH5α, the transformants were selected on a kanamycin-containing LB medium, and DNA was obtained using a DNA-spin plasmid DNA purification kit (iNtRON). The vector for the purpose of introducing the A42V mutation into the ilvN gene was named pDC24-ilvN(A42V).

**[Table 5]**

| Primer | Nucleotide sequence |
|---|---|
| SEQ ID NO: 7 | cggggatcccccgggAGGACGGTACTCAAATACTAAACTTC |
| SEQ ID NO: 8 | TGCCGAGTGTTTCGGTCTTTACAGACACGAGGGACACG |
| SEQ ID NO: 9 | TGTCTGTAAAGACCGAAACACTCGGCATCAA |
| SEQ ID NO: 10 | cggggatcccccgggGACAACTACATTATTATTATACCACA |

Thereafter, wild-type *Corynebacterium glutamicum* ATCC14067 was transformed with the pDC24-ilvN(A42V) by homologous recombination on the chromosome (van der Rest et al., Appl Microbiol Biotechnol 52:541-545, 1999). A strain in which the vector was inserted into the chromosome by recombination of homologous sequences was selected on a medium containing 25 mg/L of kanamycin. Thereafter, for the *Corynebacterium glutamicum* transformant in which the secondary recombination was completed, a gene fragment was amplified through PCR using a primer pair of SEQ ID NO: 6 and SEQ ID NO: 9, and then the strain into which the mutation was inserted was confirmed through gene sequence analysis. The recombinant strain was named *Corynebacterium glutamicum* CJ7V.

Finally, a strain was prepared by transforming the *Corynebacterium glutamicum* CJ7V with a vector in the same manner as in Example 3-1, and was named *Corynebacterium glutamicum* CJ7V::ilvB(H150R). In order to compare the L-valine production ability of the prepared strain, culturing was performed in the same manner as in Example 3-1, the L-valine concentration was analyzed, and the analyzed L-valine concentration is shown in Table 6 below.

**[Table 6]**

| L-valine production ability of CJ7V and CJ7V::ilvB(H150R) | | | | |
|---|---|---|---|---|
| Strain | L-valine (g/L) | | | |
| | Batch 1 | Batch 2 | Batch 3 | Average |
| CJ7V | 2.4 | 2.2 | 2.1 | 2.2 |
| CJ7V::*ilvB*(H150R) | 2.9 | 2.8 | 2.8 | 2.6 |

As a result, it was confirmed that the L-valine production ability of the CJ7V::ilvB(H150R) strain increased by 18% compared to that of the CJ7V strain.

### Example 3-3: Preparation of a strain into which an ilvB mutation is introduced in the Corynebacterium glutamicum CJ8V strain, and evaluation of L-valine production ability

To confirm whether the effect of increasing L-valine production ability is also exhibited in other L-valine-producing strains belonging to *Corynebacterium glutamicum*, a strain with improved L-valine production ability was constructed by introducing a mutation [ilvN(A42V); Biotechnology and Bioprocess Engineering, June 2014, Volume 19, Issue 3, pp 456-467] into *Corynebacterium glutamicum* ATCC13869.

Specifically, the above-prepared vector pDC24-ilvN(A42V) in Example 3-2 was transformed into a wild-type *Corynebacterium glutamicum* ATCC13869 strain to induce homologous recombination on the chromosome (van der Rest et al., Appl Microbiol Biotechnol 52:541-545, 1999). A strain in which the vector was inserted into the chromosome by recombination of homologous sequences was selected on a medium containing 25 mg/l of kanamycin. For the selected *Corynebacterium glutamicum* transformants, a gene fragment was amplified through PCR using a primer pair having the sequences of SEQ ID NOs: 11 and 12, and then it was confirmed through gene sequence analysis that the mutation was properly introduced. The recombinant strain was named *Corynebacterium glutamicum* CJ8V. The sequences of the primers used in the present Example are listed in Table 7 below.

**[Table 7]**

| Primer | Nucleotide sequence |
|---|---|
| SEQ ID NO: 11 | CCGCGTCACCAAAGCGGA |
| SEQ ID NO: 12 | TTAGATCTTGGCCGGAGCCA |

Finally, a strain was prepared by transforming the *Corynebacterium glutamicum* CJ8V with a vector in the same manner as in Example 3-1, and was named *Corynebacterium glutamicum* CJ8V::ilvB(H150R). In order to compare the L-valine production ability of the prepared strain, culturing was performed in the same manner as in Example 3-1, the L-valine concentration was analyzed, and the analyzed L-valine concentrations are shown in Table 8 below.

**[Table 8]**

| L-valine production ability of CJ8V and CJ8V::ilvB(H150R) | | | | |
|---|---|---|---|---|
| Strain | L-valine (g/L) | | | |
| | Batch 1 | Batch 2 | Batch 3 | Average |
| CJ8V | 2.0 | 1.8 | 1.8 | 1.9 |
| CJ8V::*ilvB*(H150R) | 2.4 | 2.3 | 2.2 | 2.3 |

As a result, it was confirmed that the L-valine production ability of the CJ8V::ilvB(H150R) strain increased by 21% compared to that of the CJ8V strain.

### Example 4. Preparation of the KCCM11201P strain into which amino acid substitution mutations other than H150R were introduced and confirmation of valine production ability

The following experiment was performed to confirm that the position of the 150th amino acid of the ilvB protein of SEQ ID NO: 1 is an important position for substitution mutation to improve valine production ability. In order to mutate histidine, which is the 150th amino acid of ilvB, to an amino acid other than arginine, site-directed mutagenesis was performed using the pDC24-ilvB(H150R) used in Example 3-1 as a template. The site-directed mutagenesis was performed by the method below.

**[Table 9]**

| PCR composition for Site-Directed Mutagenesis | |
|---|---|
| Component | Unit (µl) |
| 10X pfu-X Buffer | 5 |
| 10mM dNTP Mix | 1 |
| pfu-X Polymerase | 1 |
| Mutagenic forward primer(5 pmol) | 2 |
| Mutagenic reverse primer(5 pmol) | 2 |
| pDC24-ilvB(H150R) (template DNA, 200 ng/µl) | 1 |
| dH2O | 38 |
| Total | 50 |

**[Table 10]**

| PCR cycle for Site-Directed Mutagenesis | | |
|---|---|---|
| Cycle | Temperature | Time |
| 1 | 95 °C | 1 min |
| 18 | 95 °C | 50 sec |
| | 60 °C | 50 sec |
| | 68 °C | 9 min |
| 1 | 68 °C | 7 min |

In order to substitute histidine, which is the 150th amino acid of ilvB, with proline (P) (SEQ ID NO: 13) or asparagine (N) (SEQ ID NO: 14), which are amino acids other than arginine, PCR mixtures as shown in Table 9 above were prepared using the respective mutagenic primer sets shown in Table 11 below, and PCR was performed with the cycles shown in Table 10 above. After PCR was completed, ligation was performed using an In-Fusion Cloning Kit (Takara Bio Inc., Otsu, Japan), followed by transformation into E. coli DH5α, and it was confirmed through sequencing that the target residues in the obtained pDC24_ilvB variant plasmids were replaced with the respective mutations shown in Table 11 below.

**[Table 11]**

| Mutagenic primer set for preparing an amino acid variant plasmid at position 150 of the ilvB amino acid sequence | | |
|---|---|---|
| Variant ilvB plasmid | SEQ ID NO | Sequence (5'-3') |
| pDC24_ilvB(H150P) | 15 | TGACCAAGcctAACTTCATGGTCACCGA |
| | 16 | ATGAAGTTaggCTTGGTCACTGGCATGG |
| pDC24_ilvB(H150N) | 17 | TGACCAAGaatAACTTCATGGTCACCGA |
| | 18 | ATGAAGTTattCTTGGTCACTGGCATGG |

After transforming the pDC24_ilvB(H150P) and pDC24_ilvB(H150N) vectors prepared as shown in Table 11 into KCCM11201P by electroporation, respectively, 18 strains into which the variant ilvB gene was inserted on the chromosome were obtained through a secondary crossover process. The genetic modifications were confirmed through genome sequencing and a PCR method using primers of SEQ ID NO: 5 and SEQ ID NO: 6, which can respectively amplify the external parts of the upstream region and downstream region of homologous recombination where the corresponding gene was inserted.

The transformed strains thus obtained were named KCCM11201P::ilvB(H150P) and KCCM11201P::ilvB(H150N), respectively.

To determine the valine production of the KCCM11201P::ilvB(H150R), KCCM11201P::ilvB(H150P), and KCCM11201P::ilvB(H150N) strains prepared in Example 3-1, the strains were cultured in the same manner as in Example 3-1, the L-valine concentrations were analyzed, and the results are shown in Table 12 below.

**[Table 12]**

| L-valine production ability of a strain in which the amino acid at position 150 of the ilvB amino acid sequence is substituted with a variant | | | |
|---|---|---|---|
| | Strain Name | L-valine (g/L) | Increase compared to control |
| Control | KCCM11201P | 2.5 | - |
| Experimental group | KCCM11201P::ilvB(H150R) | 3.1 | 24.00% |
| | KCCM11201P::ilvB(H150P) | 2.7 | 8.33% |
| | KCCM11201P::ilvB(H150N) | 2.6 | 4.00% |

As may be confirmed in Table 12 above, it was confirmed that the microorganism into which a genetic mutation substituting the 150th amino acid of ilvB with another amino acid was introduced showed increased valine production compared to the control (KCCM11201P).

From the above description, those skilled in the art to which the present disclosure pertains will understand that the present disclosure may be embodied in other specific forms without departing from the technical spirit or essential characteristics thereof. In this regard, it should be understood that the embodiments described above are illustrative in all respects and not restrictive. The scope of the present disclosure should be interpreted such that all changes or modifications derived from the meaning and scope of the claims to be described later and their equivalent concepts are included within the scope of the present disclosure, rather than being limited by the detailed description above.

## Claims

1. A polypeptide in which the amino acid corresponding to the 150th residue from the N-terminus in the amino acid sequence of SEQ ID NO: 1 is substituted with another amino acid.

2. The polypeptide according to claim 1, wherein the amino acid corresponding to the 150th residue from the N-terminus in the amino acid sequence of SEQ ID NO: 1 is substituted with an amino acid selected from the group consisting of arginine, proline, asparagine, lysine, aspartic acid, glutamic acid, serine, threonine, glutamine, cysteine, glycine, alanine, valine, isoleucine, leucine, methionine, phenylalanine, tyrosine, and tryptophan.

3. The polypeptide according to claim 1, wherein the amino acid corresponding to the 150th residue from the N-terminus in the amino acid sequence of SEQ ID NO: 1 is substituted with an amino acid selected from the group consisting of arginine, proline, and asparagine.

4. The polypeptide according to claim 1, wherein the polypeptide has acetohydroxy acid synthase large subunit activity.

5. The polypeptide according to claim 1, wherein the polypeptide comprises an amino acid sequence having a homology of 90% or more with the amino acid sequence of SEQ ID NO: 1, and the amino acid corresponding to the 150th residue from the N-terminus in the amino acid sequence of SEQ ID NO: 1 is substituted with another amino acid.

6. The polypeptide according to claim 1, wherein the polypeptide comprises the amino acid sequence of SEQ ID NO: 3, SEQ ID NO: 13, or SEQ ID NO: 14.

7. A polynucleotide encoding the polypeptide according to any one of claims 1 to 6.

8. A valine-producing microorganism comprising at least one selected from the group consisting of a polypeptide in which the amino acid corresponding to the 150th residue from the N-terminus in the amino acid sequence of SEQ ID NO: 1 is substituted with another amino acid; a polynucleotide encoding the polypeptide; and a recombinant vector comprising the polynucleotide.

9. The microorganism according to claim 8, wherein the microorganism has an increased valine production ability.

10. The microorganism according to claim 8, wherein the microorganism is a microorganism of the genus *Corynebacterium*.

11. The microorganism according to claim 10, wherein the microorganism of the genus *Corynebacterium* is *Corynebacterium glutamicum*.

12. A composition for producing valine comprising the microorganism according to any one of claims 8 to 11.

13. A method for producing valine, comprising culturing the microorganism according to any one of claims 8 to 11 in a medium.

14. The method for producing valine according to claim 13, further comprising, after the culturing step, recovering valine from the cultured microorganism, the medium, or a combination thereof.

15. A method for increasing valine production, comprising culturing the microorganism according to any one of claims 8 to 11 in a medium.
